(19) Europäisches Patentamt
European Patent Office
Office européen des brevets

(11) Veröffentlichungsnummer: **0 461 503 A1**

(12) # EUROPÄISCHE PATENTANMELDUNG

(21) Anmeldenummer: **91108992.8**

(51) Int. Cl.5: **A61B 5/0275**

(22) Anmeldetag: **01.06.91**

(30) Priorität: **14.06.90 DE 4019025**

(43) Veröffentlichungstag der Anmeldung: **18.12.91 Patentblatt 91/51**

(84) Benannte Vertragsstaaten: **AT BE CH DE DK ES FR GB GR IT LI LU NL SE**

(71) Anmelder: **BAYER AG**
**W-5090 Leverkusen 1 Bayerwerk(DE)**

(72) Erfinder: **Gross, Rainer, Prof. Dr.**
**Platzhoffstrasse 23**
**W-5600 Wuppertal 1(DE)**
Erfinder: **Paffhausen, Wolgang, Dr.**
**Dürscheider Weg 17**
**W-5090 Leverkusen 3(DE)**
Erfinder: **Schade, Andreas, Dipl.-Ing.**
**Krümmgensfeld 1B**
**W-4300 Essen 14(DE)**
Erfinder: **Heusch, Gerd, Dr. Prof.**
**Einsteinstrasse 142**
**W-4040 Neuss(DE)**

(54) **Verfahren zur Durchblutungsmessung mittels nicht radioaktiver Mikrokugeln.**

(57) Es wird ein verbessertes Verfahren zur Durchblutungsmessung mittels gefärbter Mikrokugeln vorgestellt, das bisherige Auszählverfahren bei der Anwendung gefärbter Mikrokugeln durch eine schnell reproduzierbare Meßmethode ersetzt. Die aufwendige und teure Verwendung radioaktiv markierter Mikrokugeln wird dadurch vermieden. Gleichzeitig wird ein Färbeverfahren zur Präparation der Mikrokugeln vorgestellt, das sich durch eine homogene und intensive Färbung der Mikrokugeln auszeichnet.

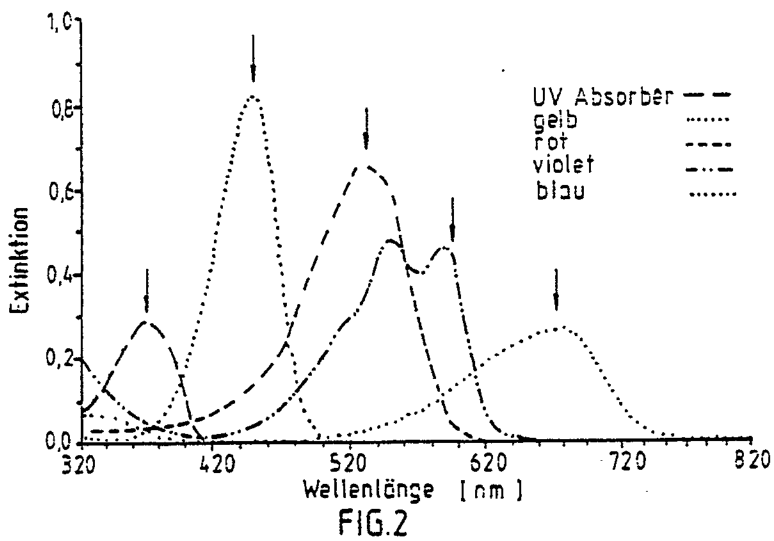

FIG.2



Rank Xerox (UK) Business Services

Die Erfindung betrifft ein Verfahren zur Messung der Durchblutung von Organ- bzw. Gewebeproben unter Durchführung der folgenden Verfahrensschritte:

1. Anfärben kleiner Kunststoffkugeln
2. Einbringen der Kunststoffkugeln in den Blutkreislauf
3. Entnahme mehrerer Gewebe- und/oder Blutproben
4. Bestimmung der Zahl der Kunststoffkugeln in den entnommenen Proben
5. Bestimmung der Durchblutung unter Verwendung der durch Schritt 4 erhaltenen Werte.

Weiter betrifft die Erfindung ein Färbeverfahren zur Anfärbung der Mikrokugeln, die in dem obengenannten Verfahren verwendet werden.

Das bekannte Prinzip der Durchblutungsmessung basiert auf der Injektion markierter Kunststoffkugeln (im folgenden auch Microspheres genannt) in die Blutbahn eines Versuchstieres, der anschließenden Entnahme von Blut- oder Gewebeproben und der Bestimmung der Zahl der Mikrokugeln in den einzelnen Proben. Aus der Konzentration der Mikrokugeln in den Gewebe- und Blutproben und der injizierten Menge an Mikrokugeln läßt sich dann die Durchblutung der untersuchten Probe zur Zeit der Injektion der Mikrokugeln bestimmen.

Bekannt ist die Durchführung dieses Verfahrens unter Verwendung radioaktiv-markierter Microspheres. Die Markierung geschieht dabei durch das Einbringen verschiedener radioaktiver Isotope in das Material, aus dem die Kugeln bestehen. Obwohl sich diese Variante der Markierung als Standardmethode etabliert hat, weist sie doch einige gravierende Nachteile auf. Insbesondere zwingt die Verwendung radioaktiver Marker den Anwender zur Einhaltung der diversen Strahlenschutzbestimmungen. So müssen die Labors, in denen Durchblutungsmessungen auf diese Art und Weise durchgeführt werden, entsprechend ausgerüstet sein und eine Kontamination der an den Versuchen beteiligten Personen vermieden werden. Ebenso müssen die Versuchstiere in entsprechend kontrollierten Ställen untergebracht werden. Dies erfordert umfangreiche bauliche Maßnahmen und schränkt somit die Zahl der Laboratorien, in denen dieses Verfahren durchgeführt werden kann, ein. Zur Verteuerung des bekannten Verfahrens trägt weiterhin der Umstand bei, daß die im Verlaufe dieses Verfahrens kontaminierten Tierkadaver in Abklinganlagen zwischengelagert werden müssen, bevor sie endgültig beseitigt werden können. Teuer und selten sind ebenfalls die nutzbaren Isotope zur Markierung, deren zum Teil kurze Halbwertzeiten die Anwendung des Verfahrens in manchen Fällen beschränken. Die Anlagen zur Messung der Radioaktivität (Gammazähler etc.) sind ebenfalls verhältnismäßig aufwendig und teuer. Zudem besitzen die radioaktiv-markierten Microspheres eine Dichte (1,3 g/ml), die von der Dichte des Blutes abweicht, so daß sich die Microspheres unphysiologisch im Blutstrom verteilen.

Ein Verfahren, bei dem die radioaktiv-markierten Mikrokugeln durch mit Farbstoff angefärbte ersetzt werden, beschreibt die europäische Anmeldung EP-0 194 517-A1. In dem beschriebenen Verfahren werden die Microspheres in einer Lösung, bestehend aus dem Salz fettlöslicher Farbstoffe und Chloroform, angefärbt. Die Auswertung erfolgt durch das Auszählen mit dem Auge, wobei die Mikrokugeln entweder direkt in der Probe oder nach Trennung von der Probe und Einbringen in ein spezielles Zählgerät, dem Hämozytometer, ausgezählt werden. Dieses Auswerteverfahren mit dem Auge macht die Methode extrem arbeitsintensiv und fehlerträchtig und verhindert eine Automation der Auswertung. Daneben besitzt das Verfahren weitere Nachteile. So weicht der Durchmesser einzelner Kugeln aus einer Probe mit bestimmtem Nenndurchmesser um bis zu 50 % von diesem Nenndurchmesser ab. Ein Fehler dieser Größe wirkt sich nachteilig auf die Genauigkeit der gesamten Messung aus.

Es bestand daher die Aufgabe, das Verfahren zur Durchblutungsmessung unter Verwendung gefärbter Mikrokugeln so zu verbessern, daß sich die Messung schnell, genau und ohne großen Aufwand, insbesondere ohne großen Aufwand an Personal durchführen lassen, Insbesondere bestand die Aufgabe darin, das Verfahren zur Bestimmung der Anzahl der Mikrokugeln in den Gewebeproben zu verbessern.

Die Erfindung, wie sie durch die Patentansprüche gekennzeichnet ist, löst die Aufgabe dadurch, daß die folgenden Verfahrensschritte durchgeführt werden:

A) Verwendung monodisperser Microspheres

B) homogene Anfärbung der Microspheres

C) Isolierung der in den einzelnen Proben enthaltenen Microspheres durch Auflösung der Gewebe- oder Blutanteile der Probe, wobei die Microspheres und deren Farbgehalt nahezu unverändert bleibt, und anschließende Abtrennung der flüssigen Phase

D) Bestimmung der Zahl der Microspheres in der Probe durch Elution des Farbstoffes und anschließender Bestimmung der eluierten Farbstoffmenge.

Das neue Verfahren besitzt den Vorteil, daß die Bestimmung der Anzahl Microspheres in den einzelnen Gewebeproben nicht mehr durch Zählen mit dem Auge durchgeführt werden muß, sondern durch ein Meßverfahren ersetzt wird. Die Bestimmung der eluierten Farbstoffmenge geschieht durch an sich bekannte

chromatographische oder spektrophotometrische Verfahren wie der Gelchromatographie oder der Absorptionsspektroskopie.

Eine bevorzugte spektroskopische Methode ist dabei die Absorptionsspektroskopie, die sich ohne größeren meßtechnischen Aufwand schnell und bequem durchführen läßt. Bei bekanntem Absorptionsverhalten eines Farbstoffes läßt sich die zu bestimmende Farbstoffmenge einer Probe auf einfache Weise durch die Messung des Absorptionsspektrums dieser Probe bestimmen, Mit üblichen Absorptionsspektrometern lassen sich Absorptionsmessungen im Sekundenbereich oder darunter durchführen, so daß mit verhältnismäßig geringem Zeitaufwand eine Vielzahl von Messungen durchführbar ist. Weiter besitzt das neue Verfahren gegenüber dem bekannten den Vorteil, daß zur Messung die gesamte Farbstoffmenge aller in einer Probe enthaltenen Mikrokugeln benutzt wird, während man bei der Auszählung einer Probe mit dem Auge aufgrund der großen Zahl von Mikrokugeln innerhalb der Probe (mehrere tausend) aus praktischen Gründen auf die Entnahme einer Stichprobe beschränkt ist. Durch diese Beschränkung wird die Genauigkeit des herkömmlichen Verfahrens weiter verschlechtert.

Es zeigt sich weiterhin, daß die Durchführung des neuen Verfahrens dadurch wesentlich verbessert wird, daß monodisperse und homogen angefärbte Mikrokugeln verwendet werden. Eine monodisperse Probe von Mikrokugeln im Sinne der Erfindung liegt dann vor, wenn der Durchmesser der einzelnen Kugeln um weniger als 10 % vom Nenndurchmesser der Probe abweicht. Eine homogene und intensive Färbung der Mikrokugeln im Sinne der Erfindung liegt dann vor, wenn sich zwei beliebige Stichproben von etwa 1000 Kugeln aus der Gesamtmenge der gefärbten Kugeln (etwa $10^8$) in ihrem Farbstoffanteil, d.h. in der Absorption der aus ihnen eluierten Farbstoffmenge um höchstens 3 % unterscheiden, Die Microspheres sind dann genügend intensiv gefärbt, wenn die Farbstoffmenge von etwa 1000 Kugeln eine Extinktion von $\geq$0,1 ergibt. Die Nachweisgrenze liegt bei einer Extinktion von etwa 0,005.

Eine bevorzugte Durchführungsform des neuen Verfahrens ist dadurch gekennzeichnet, daß verschiedene Chargen von Microspheres mit spektral unterscheidbaren Farbstoffen angefärbt werden und diese unterschiedlich gefärbten Microspheres in verschiedene Gefäßgebiete oder zu unterschiedlichen Zeitpunkten in die Blutbahn injiziert werden.

Da man bei Durchblutungsmessungen beispielsweise die Durchblutung eines Gewebes vor, während und nach der Beseitigung einer herbeigeführten Schädigung oder den Effekt von physiologischen oder pharmakologischen Einflüssen auf die Durchblutung untersuchen möchte, ist die Verwendung verschieden gefärbter Microspheres besonders vorteilhaft, Durch die Verwendung unterschiedlicher Farben für die Injektion zu unterschiedlichen Zeitpunkten und/oder an unterschiedlichen Injektionsstellen ist es möglich, ein zeitlich oder örtlich differenziertes Bild von der Durchblutung eines Gewebes zu erhalten. Die Anwendung unterschiedlich gefärbter Microspheres ist durch das in EP-0 194 517-A1 beschriebene Verfahren bekannt, sie ist jedoch durch das oben beschriebene nachteilige Auszählverfahren mit dem Auge deutlich erschwert, insbesondere dann, wenn die unterschiedlich gefärbten Kugeln innerhalb einer Probe mit deutlich verschiedenen Konzentrationen vorliegen. Aus dem Absorptionsspektrum einer Lösung, in der unterschiedliche Farbstoffe vorliegen, lässen sich jedoch unter der Voraussetzung, daß die Einzelspektren der jeweiligen Farbstoffe bekannt sind, durch an sich bekannte Verfahren, wie beispielsweise durch eine Matrixinversionstechnik (Computer Programs in Biomedicine 9, 1979, 19-38), die Konzentration der einzelnen Farbstoffe bestimmend Voraussetzung dazu ist jedoch, daß die Absorptionsmaxima der einzelnen Farbstoffe nicht vollständig überlappen. Weiter muß die Farbstärke während der Verweilzeit im Blut bzw. im Gewebe und während des Auflösens von Blut bzw. Gewebe nahezu erhalten bleiben, d.h. die Farbstärke der isolierten Mikrokugeln darf sich um höchstens 3 % von der der ursprünglich gefärbten Mikrokugeln unterscheiden.

Eine weitere bevorzugte Durchführungsform des neuen Verfahrens ist dadurch gekennzeichnet, daß monodisperse Microspheres mit einem Nenndurchmesser aus dem Bereich zwischen 7 $\mu$m bis 30 $\mu$m, bevorzugt zwischen 10 $\mu$m bis 15 $\mu$m, und einer Toleranz von maximal 10 % verwendet werden.

Mikrospheres der bevorzugten Größe lassen sich besonders vorteilhaft bei der Durchblutungsmessung verwenden. Der Nenndurchmesser einer Charge von Microspheres ist dabei der mittlere Durchmesser der Microspheres dieser Charge, der aus dem angegebenen Bereich entsprechend der gewünschten Messung ausgewählt wird. Bei einer monodispersen Charge von Microspheres weichen die Durchmesser einzelner Kugeln dieser Charge um maximal 10 % vom Nenndurchmesser ab. Die Verwendung polydisperser Microspheres führt zum Verschluß sehr unterschiedlich großer Gefäße und bei zu geringen Durchmessern zu Rezirkulationsphänomenen und damit zu großen Streuungen und Meßfehlern.

Eine weitere bevorzugte Durchführungsform des neuen Verfahrens ist dadurch gekennzeichnet, daß gefärbte Microspheres mit einer spezifischen Dichte verwendet werden, die nahe an der Dichte von Blut liegt, vorzugsweise zwischen 1,00 g/ml und 1,10 g/ml, insbesondere bevorzugt zwischen 1,04 g/ml und 1,06 g/ml.

Die Verwendung von Kugeln, deren spezifische Dichte sich nur wenig von der Dichte der Bestandteile

des Blutes unterscheiden, vermögen dem Blutstrom besser zu folgen als Kugeln gleicher Abmessung, deren spezifisches Gewicht deutlich höher liegt. Bei der Wahl des Materials, aus dem die Mikrokugeln hergestellt werden, sind deshalb Stoffe bevorzugt, deren Dichte in dem angegebenen Bereich liegen. Als besonders geeignet erweist sich Polystyrol.

Ein Färbeverfahren für Mikrokugeln aus Polystyrol zur Verwendung in dem neuen Verfahren zur Messung der Durchblutung ist dadurch gekennzeichnet, daß zum Färben Farbstoffe ohne salzbildende Gruppen, insbesondere Dispersionsfarbstoffe, verwendet werden.

Es hat sich überraschend erwiesen, daß sich die Microspheres auch mit üblichen Dispersionsfarbstoffen färben lassen, so daß auf die Verwendung salzbildender, fettlöslicher und wasserunlöslicher Farben, die in dem durch EP-0 194 517-A1 bekannten Verfahren ausschließlich verwendet werden, verzichtet werden kann. Mit der Verwendung der neuen Farbstoffe wird sogar eine intensivere und homogenere Anfärbung der Microspheres erzielt. Dabei ergaben Dispersionsfarbstoffe auf der Basis von Azoverbindung, Anthrachinonverbindung und insbesondere Fluoreszenzfarbstoffe auf Cumarinbasis besonders gute Ergebnisse.

Im folgenden wird das neue Verfahren mit Hilfe der Zeichnung beispielhaft erläutert, Es zeigt:

Fig. 1 ein zusammengesetztes Spektrum einer Probe, in der 5 Farbstoffe enthalten sind.

Fig. 2 das Spektrum nach Fig. 1, zerlegt in die Einzelspektren der Farbstoffe.

Fig. 3 ein Vergleich des neuen Verfahrens (Colored Microspheres) mit dem Standardverfahren - (Radioactive Microspheres), vorgenommen an 4 Schweinen mit 1080 Datenpunkten.

Fig. 4 Vergleich des neuen Verfahrens (Colored Microspheres) mit dem Standardverfahren (Radioactive Microspheres), vorgenommen an 4 Hunden mit 1813 Datenpunkten.

Zur beispielhaften Durchführung des Verfahrens wurden Polystyrolmikrokugeln mit einem Durchmesser von 10 µm und 15 µm und einem Variationskoeffizienten von jeweils maximal 5 % verwendet.

Diese Microspheres wurden nach einer Reinigung mit 70 %igem Ethanol und anschließender Trocknung mit jeweils einem der folgenden Farbstoffe gefärbt:

1. UV-Absorber

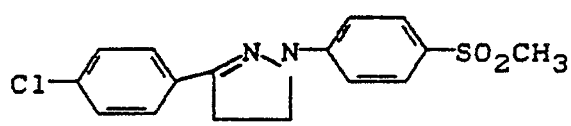

2. gelb

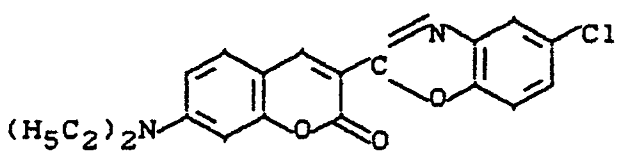

3. rot-violett

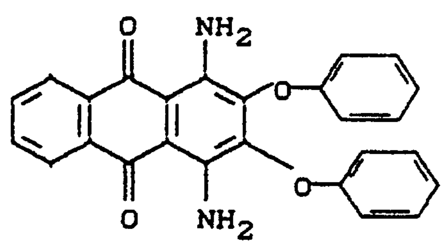

4. blau

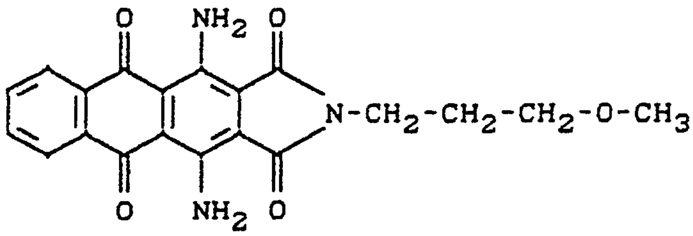

5. rot

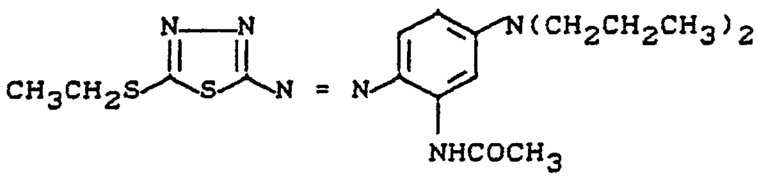

Zur Färbung mit dem roten und dem violetten Farbstoff wurden die Microspheres einer Lösung zugegeben, die aus 400 mg des entsprechenden Farbstoffes, 2 ml destillierten Wassers, 1 ml einer 1 %igen wäßrigen Lösung aus 65 % Dichlortoluol, 20 % O-Kresotinsäuremethylester und 15 % Emulgator aus 55 % Rizinusöl und 45 % des Monoethanolaminsalzes der Marlon AS3 Säure bestand.

Zur Färbung mit dem UV-Absorber wurden die Microspheres 10 ml einer Mischung von 85 Vol.-% Toluol und 15 Vol.-% Dimethylformamid zugegeben, die zuvor mit dem Farbstoff bei 80° C gesättigt worden war.

Der blaue und der gelbe Farbstoff wurden zunächst durch Rekristallisation in Dichlormethan vorgereinigt. Danach wurden die Microspheres zu einer Mischung von 100 mg gereinigtem Farbstoff und 1 ml 1,2,4-Trichlorbenzol zugegeben.

Alle Mischungen aus Microspheres und Farbstoffen wurden in kochendem Wasser erhitzt (5 Minuten für die Gelb- und Blaumischung, 90 Minuten für die UV-Absorber-, Rot- und Violettmischung). Danach wurden die gefärbten Microspheres durch Filtration vorgereinigt und anschließend die Farbstoffreste durch Resuspension in absolutem Ethanol, einer Ultraschallbehandlung und anschließendem Zentrifugieren bei Verwerfung des Überstandes gereinigt. Nach einer Trocknung und Resuspension in Wasser wurden die Kugeln in Portionen für den späteren Gebrauch im Experiment aufgeteilt.

Zur Messung der Durchblutungsverteilung im Kreislaufversuch wurden die gefärbten Microspheres erneut mit Ethanol gewaschen und vakuumgetrocknet. Danach wurden sie in physiologische Kochsalzlösung resuspendiert und nach den bekannten Methoden dem Kreislauf zugegeben, vornehmlich nach entsprechender Vermischung im strömenden Blut in eine Organarterie oder in den linken Herzvorhof oder die linke Herzkammer. Bei der Applikation von verschieden gefärbten Microspheres wurden somit zeitlich unterschiedliche Durchblutungszustände festgehalten.

Nach Beendigung des Experiments wurden Gewebe- bzw. Blutproben in üblicher Weise gewonnen und portioniert.

Die Gewebeproben wurden dabei auf Massen zwischen 0,3 bis 2 g portioniert. Die Proben wurden in 4-molarer Kaliumhydroxidlösung, die 2 % Tween-80 enthielt, in einem Glasröhrchen bei 72° C aufgelöst. Anschließend wurde die Lösung durch einen Mikrofilter abgesaugt. Die zurückbleibenden Microspheres wurden zusammen mit den Filtern gewaschen und getrocknet.

Zur Elution des Farbstoffes wurden die Filter mitsamt der Mikrokugeln in ein Zentrifugenröhrchen überführt und die Farbstoffe durch Zugabe von 100 µl Dimethylformamid herausgelöst. Die Farbstofflösung wurde anschließend für 5 Minuten bei 2000 g zentrifugiert und in ein kleines Glasröhrchen überführt. Diese Lösung wurde durch nochmalige Zentrifugation (3 Minuten, 2000 g) von verbleibenden Partikeln gereinigt.

Mit Blutproben wurde entsprechend verfahren.

Die Absorption der auf diese Weise erhaltenen Farbstofflösung wurde mit einem Dioden-Array UV/VIS Spektrophotometer mit einer spektralen Auflösung von 2 nm in einem Wellenlängenbereich von 190 bis 820 nm gemessen. Fig. 1 zeigt ein auf diese Weise erhaltenes Extinktionsspektrum einer Probe, die 5 verschiedene Farbstoffe enthielt. Fig. 2 zeigt die Einzelspektren der jeweiligen Farbstoffe, aus dem sich das Gesamtspektrum additiv zusammensetzt. Die Farbstoffe wurden bei den Wellenlängen 370 nm, 448 nm,

530 nm, 594 nm und 672 nm vermessen (angedeutet durch Pfeile in Fig. 1 und 2). Durch Integration von 100 Einzelmessungen von jeweils 0,1 sec Dauer wurde die Standardabweichung jedes einzelnen Absorptionswertes auf weniger als 0,1 % des Mittelwertes gesenkt.

Auf diese Weise konnte eine sehr große Zahl an Proben ausgewertet werden. So wurden für die Vergleichsuntersuchungen, die in Fig. 3 und 4 dargestellt sind, 1080 bzw. 1813 Proben verglichen.

Zum Vergleich wurden Schweinen und Hunden unter steadystate Bedingungen jeweils ein Paar gefärbter und radioaktiv-markierter Microspheresdispersionen injiziert. In der ersten Versuchsserie bei Schweinen wurde direkt in die linke vordere Koronararterie injiziert, wobei die Koronardurchblutung durch intrakoronare Adenosininfusion bei konstantem koronarem Perfusionsdruck erhöht wurde und durch Absenkung des koronaren Perfusionsdruckes eine regionale Myokardischämie hervorgerufen wurde. In der zweiten Versuchsserie wurden radioaktive und gefärbte Microspheres Hunden intra-atrial injiziert. Aus den 1080 Proben, die bei Schweinen nach intrakoronarer Injektion gemessen wurden, ergab sich eine enge Korrelation zwischen der mit radioaktiv-markierten Microspheres und der mit gefärbten Microspheres gemessenen Myokarddurchblutung, wobei der Korrelationskoeffizient 0,98 betrug. Die lineare Regression ist mit $y = 1{,}00 \times X + 0{,}01$ nicht von der Identitätsgeraden zu unterscheiden (siehe Fig. 3).

Bei der systemischen Applikation in den Hundeversuchen wurden insgesamt 1813 Proben aufgearbeitet. Auch hier ergab sich eine enge Korrelation mit einem Korrelationskoeffizienten von 0,97, jedoch wurde hier eine systematisch niedrigere Durchblutungsbestimmung mit Hilfe der radioaktiv-markierten Microspheres als mit den gefärbten Microspheres festgestellt (Fig. 4). Die Regressionsgerade lautet $y = 0{,}92 \times X - 0{,}19$. Diese Abweichung ist wahrscheinlich eine Folge der großen spezifischen Dichte der radiaktiv-markierten Microspheres, die stark von der Dichte des Blutes und der farbig markierten Microspheres abweicht.

**Patentansprüche**

1. Verfahren zur Messung der Durchblutung von Organ- bzw. Gewebeproben unter Durchführung der folgenden Verfahrensschritte:
   1. Anfärben von Mikrokugeln (Microspheres)
   2. Einbringen der Mikrokugeln in den Blutkreislauf
   3. Entnahme mehrerer Blut- und/oder Gewebeproben
   4. Bestimmung der Zahl der Mikrokugeln in den entnommenen Proben
   5. Bestimmung der Durchblutung unter Verwendung der durch Schritt 4 erhaltenen Werte,

   gekennzeichnet durch die folgenden Schritte:
   A) Verwendung monodisperser Mikrokugeln
   B) homogene Anfärbung der Mikrokugeln
   C) Isolierung der in den einzelnen Proben enthaltenen Mikrokugeln durch Auflösung der Gewebe- oder Blutanteile der Probe, wobei die Mikrokugeln und deren Farbgehalt nahezu unverändert bleiben, und anschließende Abtrennung der flüssigen Phase
   D) Bestimmung der Zahl der Mikrokugeln durch Elution des Farbstoffes und anschließender Bestimmung der eluierten Farbstoffmenge.

2. Verfahren nach Anspruch 1, dadurch gekennzeichnet daß die eluierte Farbstoffmenge durch Absorptionsspektroskopie bestimmt wird.

3. Verfahren nach Anspruch 1, dadurch gekennzeichnet, daß verschiedene Chargen von Mikrokugeln mit spektral unterscheidbaren Farbstoffen angefärbt werden und diese unterschiedlich gefärbten Mikrokugeln in verschiedene Gefäßgebiete oder zu unterschiedlichen Zeitpunkten in die Blutbahn injiziert werden.

4. Verfahren nach den Ansprüchen 1 bis 2, dadurch gekennzeichnet, daß monodisperse Mikrokugeln mit einem Nenndurchmesser aus dem Bereich zwischen 7 µm und 30 µm, bevorzugt zwischen 10 µm und 15 µm, und einer Toleranz von maximal 10 % verwendet werden.

5. Verfahren nach den Ansprüchen 1 bis 3, dadurch gekennzeichnet, daß gefärbte Mikrokugeln mit einer spezifischen Dichte verwendet werden, die nahe an der Dichte von Blut liegt, vorzugsweise zwischen 1,00 g/ml und 1,10 g/ml, insbesondere bevorzugt zwischen 1,04 g/ml und 1,06 g/ml.

6. Färbeverfahren für Mikrokugeln aus Polystyrol zur Verwendung in dem Verfahren nach den Ansprüchen

1 bis 4, dadurch gekennzeichnet, daß zum Färben Farbstoffe ohne salzbildende Gruppen, insbesondere Dispersionsfarbstoffe, verwendet werden.

7. Verfahren nach Anspruch 5, dadurch gekennzeichnet, daß Dispersionsfarbstoffe auf der Grundlage von Azoverbindungen, Anthrachinonverbindungen oder Cumarinverbindungen, bevorzugt Fluoreszenzfarbstoffe auf Cumarinbasis verwendet werden.

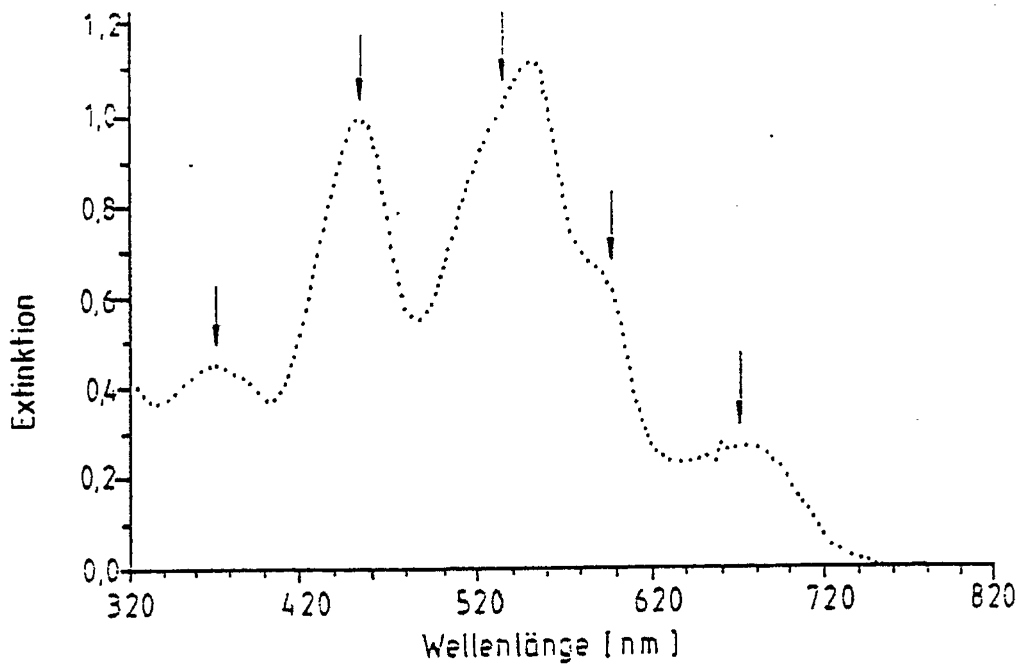
Extinktion
1,2
1,0
0,8
0,6
0,4
0,2
0,0
320
420
520
620
720
820
Wellenlänge [nm]

FIG. 1

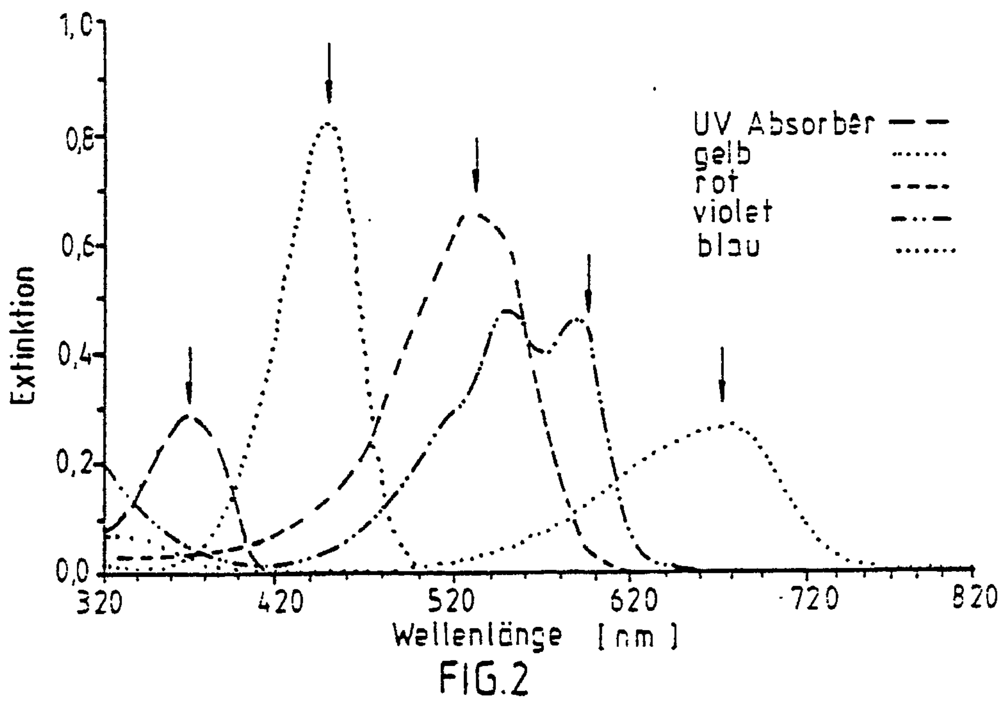
Extinktion
1,0
0,8
0,6
0,4
0,2
0,0
320
420
520
620
720
820
Wellenlänge [nm]
UV Absorber
gelb
rot
violet
blau

FIG. 2

FIG. 3

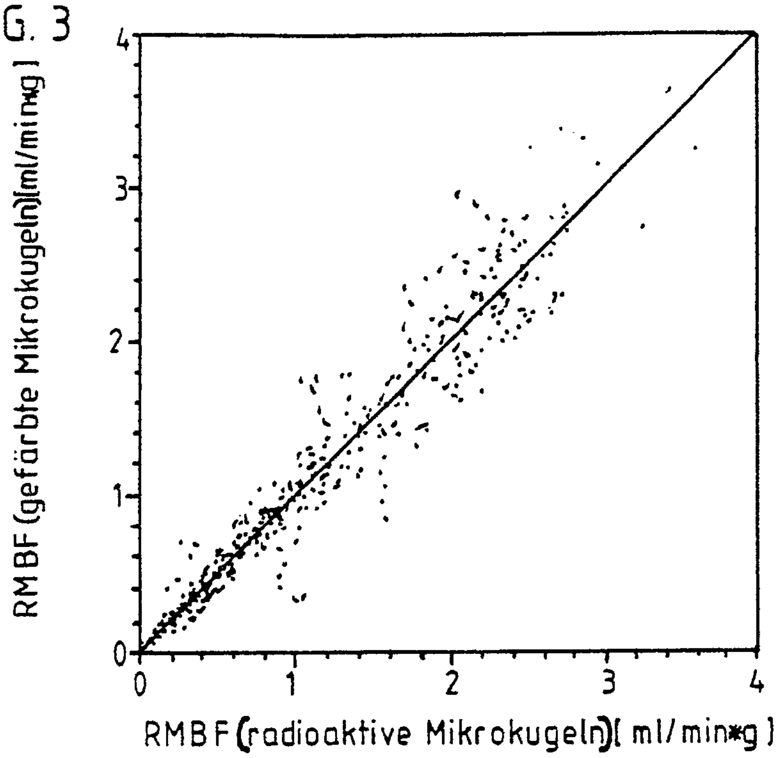
RMBF (gefärbte Mikrokugeln)[ml/min*g]
0
1
2
3
4
RMBF (radioaktive Mikrokugeln)[ml/min*g]

FIG. 4

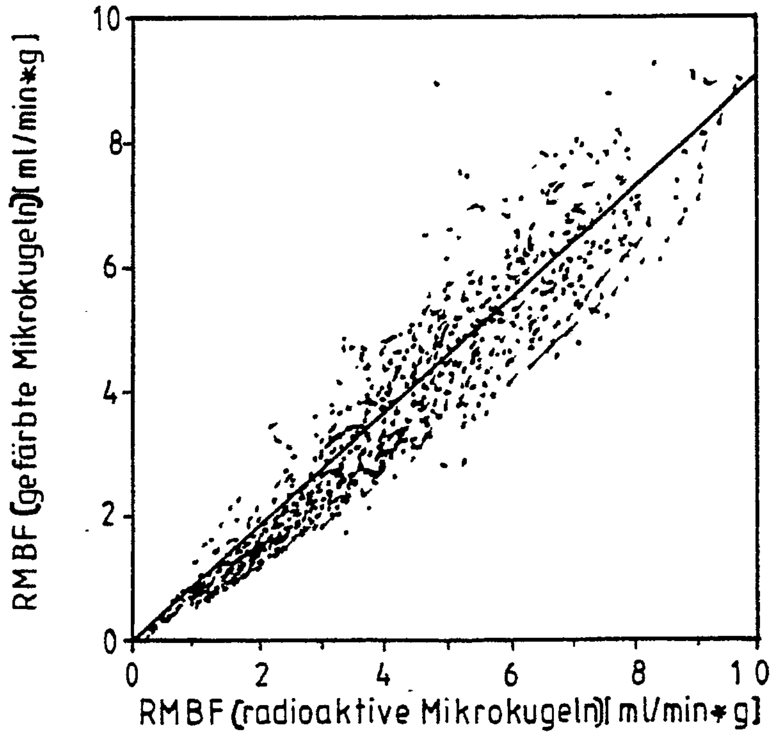
RMBF (gefärbte Mikrokugeln)[ml/min*g]
0
2
4
6
8
10
RMBF (radioaktive Mikrokugeln)[ml/min*g]

Europäisches Patentamt

# EUROPÄISCHER RECHERCHENBERICHT

Nummer der Anmeldung

**EP 91 10 8992**

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| A,D | EP-A-0 192 517 (SHELL, W.E.)<br>* Spalte 4, Zeile 54 - Spalte 7, Zeile 31 *<br>– – – | 1,4,6 | A 61 B 5/0275 |
| A | DE-A-1 498 534 (FRIED. KRUPP GMBH)<br>* Seite 1, Zeilen 3-15 *<br>– – – | 1,2 | |
| A | US-A-7 097 03 (N.H. LAZAROW)<br>* Spalte 2, Zeile 51 - Spalte 4, Zeile 32; Figuren 1,2 *<br>– – – | 1 | |
| A,D | COMPUTER PROGRAMS IN BIOMEDICINE vol. 9, 1979, Seites 11-38; R. SCHOSSER et al.: "MIC-II - a program for the determination of cardiac output, arterio - venous shunt and regional bloodflow using the radioactive microsphere methode"<br>* Seite 19, Zeile 1 - Seite 22, rechte Spalte, Zeile 23; Figuren 1,2 *<br>– – – – – | 1,2 | |

RECHERCHIERTE SACHGEBIETE (Int. Cl.5)

A 61 B 5/00

Der vorliegende Recherchenbericht wurde für alle Patentansprüche erstellt

| Recherchenort | Abschlußdatum der Recherche | Prüfer |
|---|---|---|
| Berlin | 02 September 91 | WEIHS J.A. |

KATEGORIE DER GENANNTEN DOKUMENTE

X: von besonderer Bedeutung allein betrachtet
Y: von besonderer Bedeutung in Verbindung mit einer anderen Veröffentlichung derselben Kategorie
A: technologischer Hintergrund
O: nichtschriftliche Offenbarung
P: Zwischenliteratur
T: der Erfindung zugrunde liegende Theorien oder Grundsätze

E: älteres Patentdokument, das jedoch erst am oder nach dem Anmeldedatum veröffentlicht worden ist
D: in der Anmeldung angeführtes Dokument
L: aus anderen Gründen angeführtes Dokument

&: Mitglied der gleichen Patentfamilie, übereinstimmendes Dokument